(19) Europäisches Patentamt
European Patent Office
Office européen des brevets

(11)  EP 2 893 871 A1

(12)  **EUROPEAN PATENT APPLICATION**
published in accordance with Art. 153(4) EPC

(43) Date of publication:
**15.07.2015 Bulletin 2015/29**

(51) Int Cl.:
*A61B 5/02* (2006.01)      *A61B 5/1477* (2006.01)
*G06F 19/00* (2011.01)

(21) Application number: **13835126.7**

(22) Date of filing: **02.07.2013**

(86) International application number:
**PCT/KR2013/005835**

(87) International publication number:
**WO 2014/038778 (13.03.2014 Gazette 2014/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **06.09.2012 KR 20120098774**

(71) Applicant: **Lee, Dong Hwa**
**Yongin-si, Gyeonggi-do 446-891 (KR)**

(72) Inventor: **Lee, Dong Hwa**
**Yongin-si, Gyeonggi-do 446-891 (KR)**

(74) Representative: **Pontet Allano & Associes**
**Parc Les Algorithmes, Bâtiment Platon**
**CS 70003 Saint-Aubin**
**91192 Gif-sur-Yvette Cedex (FR)**

(54)  **METHOD FOR PROCESSING BIOSIGNAL**

(57) Disclosed herein is a method for processing a physiological signal to measure physiological characteristics of a living organism. The method includes: (a) dividing $P_1'(t)$, which is obtained by differentiating one signal function $P_1(t)$ between two signal functions having different wavelength bands and representing the physiological characteristics of the living organism, by $P_2'(t)$ obtained by differentiating the other signal function $P_2(t)$; and (b) obtaining a constant b of the following function $n_1(t)$ and a cycle of the following function $s_1(t)$ through Math

Formula 1:

$$(P_1'(t)/P_2'(t))|_{t=t1, t2, t3,...} = k,$$

(where $P_1(t)=s_1(t)+n_1(t)$, $P_2(t)=s_2(t)+n_2(t)$, $as_1(t)=s_2(t)$, $bn_1(t)=n_2(t)$, a, b and k are constants, t is time, and $n_1(t)$ and $n_2(t)$ are signal functions due to noise when measuring the physiological signal).

In this method, a signal cycle of desired data on a user, that is, data from which a noise component has been removed, can be extracted through a simple process, thereby simplifying the method for detecting physiological characteristics.

【Figure 3】

## Description

[Technical Field]

The present invention relates to a method for processing a physiological signal, and more particularly, to a method for processing a physiological signal in which, when a non-invasive method is used to measure oxygen saturation, pulse or similar biological characteristics (i.e., physiological characteristics), a characteristic constant and cycle of a signal for checking the biological characteristics can be readily obtained, whereby the biological characteristics can be rapidly and easily determined.

[Background Art]

In general, a signal processor processes a physiological signal based on various physiological characteristics, for example, blood gas saturation such as oxygen saturation or the like, blood pressure, heart rate, electrocardiogram, and the like, and obtains a physiological signal by extracting or removing one of a main signal and a noise signal from a composite signal including the main and noise signal.

For instance, the composite signal includes a main signal component containing desired data on a user, and a noise signal component generated by noise. At this time, if the noise signal occupies a frequency spectrum different from that of the main signal, it is possible to remove the noise signal or extract the main signal from the whole signal through existing filtering methods such as low pass filtering, band pass filtering, high pass filtering, etc. However, the main signal and the noise signal may have an overlapping frequency spectrum.

In this case, the existing filtering methods cannot effectively extract the main signal or remove the noise signal. Further, if information about one of the two signal components is sufficient, a correlation canceller such as an adaptive noise canceller may be used to extract the signal component. However, in general, information about the signals is not sufficient.

Accordingly, in the case of physiological monitoring, i.e., in monitoring of physiological characteristics, it is not easy to determine information about the main signal component and the noise signal component. Physiological monitoring generally uses a measured signal derived from a biological system such as a human body in order to monitor physiological characteristics.

The subjects of the measurement in physiological monitoring systems generally include various types such as electrocardiographs, blood pressure, blood gas saturation (e.g.: oxygen saturation or the like), capnographs, monitoring of different blood components, heart rate, respiratory rate, etc. As an example of oxygen saturation which is a form of blood gas saturation, there is arterial oxygen saturation.

Measurements of such various physiological characteristics may be achieved using energy attenuation through an inspection medium such as a finger in the form of selected energy. Among these, blood gas monitoring is an example of physiological monitoring based on the measurement of energy attenuation due to biological tissues or materials.

In blood gas monitoring, light for measuring a physiological signal is emitted to an inspection medium, for example, a patient's finger, and an attenuation characteristics of the light are measured over time. At this time, an output signal of a blood gas monitor susceptible to flow of arterial blood includes a signal having a waveform reflecting an arterial pulse of a patient.

Examples of such physiological monitoring technology are disclosed in patent documents, such as US Patent Nos. 6,002,952 and 5,490,505. In the case of using an inspection medium such as a finger to carry out physiological monitoring, venous blood, movement of the finger and similar noise factors may cause irregular energy attenuation.

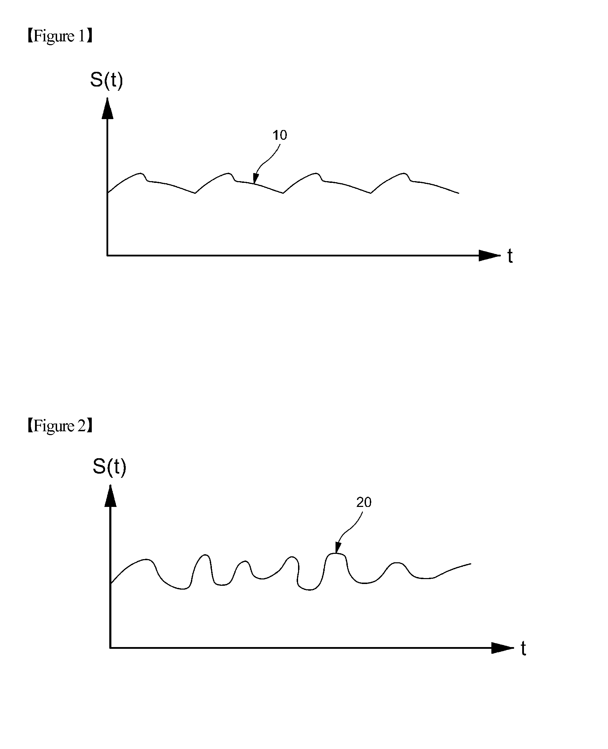

In US Patent No. 6,002,952, Fig. 1 shows a graph of a signal waveform (i.e., a waveform of the main signal) according to regular energy attenuation, and Fig. 2 shows a graph of a signal waveform according to practical irregular energy attenuation, i.e., considering a noise component.

However, if an irregular signal waveform, due to venous blood, patient movement, and the like, is detected as described above, complicated operations and processes are needed to extract a main signal from an irregular signal waveform. Therefore, it is difficult to extract information of the main signal, i.e., the cycle and characteristic constant of the main signal and to output the physiological characteristics based on the information.

[Disclosure]

[Technical Problem]

The present invention has been conceived to solve the above problems, and it is an aspect of the invention to provide a method for processing a physiological signal, in which information of a physiological signal measured by (i.e., input by) a detector, particularly, characteristics of a main signal can be obtained without noise signal through a simple process.

[Technical Solution]

In accordance with one aspect of the present invention, a method for processing a physiological signal to measure physiological characteristics of a living organism includes: (a) dividing $P_1'(t)$, which is obtained by differentiating one signal function $P_1(t)$ between two signal functions having different wavelength bands and representing the physiological characteristics of the living organism, by $P_2'(t)$ obtained by differentiating the other signal function $P_2(t)$; and (b) obtaining a constant b of the following function $n_1(t)$ and a cycle of the following function $s_1(t)$ through Math Formula 1 as follows:

[Math Formula 1]

$$(P_1'(t)/P_2'(t))|_{t=t1, t2, t3,...} = k,$$

(where $P_1(t)=s_1(t)+n_1(t)$, $P_2(t)=s_2(t)+n_2(t)$, $as_1(t)=s_2(t)$, $bn_1(t)=n_2(t)$, a, b and k are constants, t is time, and $n_1(t)$ and $n_2(t)$ are signal functions due to noise when measuring the physiological signal).

The step of obtaining a constant b of the function $n_1(t)$ and a cycle of the function $s_1(t)$ comprises obtaining the constant b of the function $n_1(t)$ and the cycle of the function $s_1(t)$ by setting the value k of Math Formula 1 such that a variable t allowing the function $P_1'(t)/P_2'(t)$ to have the same value can satisfy a condition of including a value repeated at a regular cycle.

The step of obtaining a constant b of the function $n_1(t)$ and a cycle of the function $s_1(t)$ may comprise obtaining the constant b of the function $n_1(t)$ and the cycle of the function $s_1(t)$ by setting the value k of Math Formula 1 so as to satisfy a condition that intersecting points between a transverse line (k) parallel to an axis (t) of abscissa of the function $P_1'(t)/P_2'(t)$ and the function $P_1'(t)/P_2'(t)$ include intersecting points repeated at regular intervals with at least one intersecting point therebetween.

The step of obtaining a constant b of the function $n_1(t)$ and a cycle of the function $s_1(t)$ may comprise obtaining the constant b of the function $n_1(t)$ and the cycle of the function $s_1(t)$ by setting the value k of Math Formula 1 so as to satisfy a condition that intersecting points between a transverse line (k) parallel to an axis (t) of abscissa of the function $P_1'(t)/P_2'(t)$ and the function $P_1'(t)/P_2'(t)$ include intersecting points repeated in a certain pattern.

The step of obtaining a constant b of the function $n_1(t)$ and a cycle of the function $s_1(t)$ may comprise obtaining the constant b of the function $n_1(t)$ and the cycle of the function $s_1(t)$ by setting the value k of Math Formula 1 so as to satisfy a condition that intersecting points between a transverse line (k) parallel to an axis (t) of abscissa of the function $P_1'(t)/P_2'(t)$ and the function $P_1'(t)/P_2'(t)$ include intersecting points of a preset pattern.

The Math Formula 1 used in extracting the constant b of the function $n_1(t)$ and the cycle of the function $s_1(t)$ can be obtained based on that $s_1'(t)$ obtained by differentiating the function $s_1(t)$ is 0 (zero).

The physiological characteristics may include pulse oxygen saturation, and may be measured by an infrared signal and a red light signal.

After the constant b is extracted in step (b), it is possible to extract a reference waveform of the signal function $s_1(t)$ through Math Formula 4 as follows:

[Math Formula 4]

$$P_1(t)-P_2(t)/b=s_1(t)+n_1(t)-(as_1(t)+bn_1(t))/b=(1-a/b)s_1(t).$$

[Advantageous Effects]

According to the present invention, the period of data without noise can be obtained through a simple process and detection process of the physiological characteristics can be simplified.

[Description of Drawings]

The above and/or other aspects will become apparent and more readily appreciated from the following description of exemplary embodiments, taken in conjunction with the accompanying drawings, in which:

Fig. 1 is a graph schematically showing an exemplary waveform of an ideal signal (a main signal) free from any noise factors;

Fig. 2 is a graph schematically showing an example waveform of a practical signal (a composite signal) with noise;

Fig. 3 is a flowchart showing a method for processing a physiological signal according to one embodiment of the present invention;

Fig. 4 is a graph for explaining a method of extracting a cycle of a main signal in the method for processing a physiological signal according to the embodiment of the present invention; and

Fig. 5 is a graph for explaining a method of extracting a reference pattern of a main signal in the method for processing a physiological signal according to the embodiment of the present invention.

[Best Mode]

Now, exemplary embodiments of the invention will be described in detail with reference to the accompanying drawings. In describing the exemplary embodiments, like terms and numerals refer to like components throughout the specification and the accompanying drawings, and thus repeated descriptions thereof will be omitted.

Referring to Figs. 1 and 2, when a non-invasive method is used to measure physiological characteristics, a waveform 10 of an ideal signal (i.e., a main signal) at a regular cycle is detected, as shown in Fig. 1, if there is no noise factor. On the other hand, if a noise factor is present, an irregular waveform 20 of a signal in which noise signal is mixed with the main signal is detected, as shown in Fig. 2.

The irregular signal waveform 20 shows a result of adding a noise factor to the ideal signal waveform 10. If such a noise factor is present in measurement of the physiological characteristics of a living organism, it can be difficult to distinguish the main signal having data of the physiological characteristics even though the noise factor is small.

To measure the physiological characteristics, signals detected by a sensor such as a pulse oximeter or the like, that is, measured signals, include two measured signals. The two measured signals may belong to different wavelength bands, and thus the physiological characteristics of the living organism may be determined through the two signal components.

Each of the two measured signals, that is, the signals 20 (measured signals or input signals) measured by the sensor, includes a main signal 10 and a noise signal. Here, the main signal is an ideal measured signal when no noise factor is present, and the noise signal is a signal measured due to a noise factor, such as movement of a patient, venous blood, etc.

As detailed examples of the two measured signals, there are signals used in measuring the oxygen saturation, which includes infrared signals measured using infrared (IR) light and red light signals measured using red light.

Here, a device (i.e., a sensor) for measuring the oxygen saturation of arterial blood through a non-invasive method is called a pulse oximeter, which obtains blood oxygen saturation, which is an indicator of the amount of hemoglobin present in the arterial blood. The oxygen saturation is information utilized in heart output or pulmonary function evaluation, organ specific perfusion or cardiovascular state evaluation, hypoxia diagnosis, etc., which is applied to various fields including not only general medicine but also sports science.

The pulse oximeter includes two light emitters for emitting two kinds of light to a body (e.g., a finger) of a patient (e.g., an examinee), and a detector such as a photodiode, in which two light emitters are installed, for example, a red light emitting diode (LED) for emitting red light and an infrared LED for outputting infrared light may be provided.

Further, each of the two measured signals exhibiting the foregoing oxygen saturation or similar physiological characteristics is a composite signal wherein a main signal and noise signal are mixed, as described above. Hereinafter, a function representing the waveform 10 of the main signal will be denoted by $s(t)$, a function representing the waveform 20 of the noise signal (i.e., a signal with noise in measurement of the physiological signals) will be denoted by $n(t)$, and a function of the measured signals (i.e., the composite signals) in this embodiment will be denoted by $P(t)$ which is represented by the sum of the functions $s(t)$ and $n(t)$.

In more detail, one of two signals, i.e., the two measured signals representing the physiological characteristics of a living organism such as a human body may be represented by the function $P_1(t)$, and the other signal may be represented by the function $P_2(t)$. The functions $P_1(t)$ and $P_2(t)$ are not limited to a certain signal. In other words, if one of the two signals is $P_1(t)$, the other signal is $P_2(t)$.

Here, $P_1(t)$ and $P_2(t)$ are defined as follows.

$$P_1(t)=s_1(t)+n_1(t), \; P_2(t)=s_2(t)+n_2(t)$$

In $P_1(t)$ and $P_2(t)$, as $s_1(t)=s_2(t)$ and $bn_1(t)=n_2(t)$, and a and b are constants. Also, $P_1(t)$ and $P_2(t)$ are time dependent functions, in which a variable t refers to time.

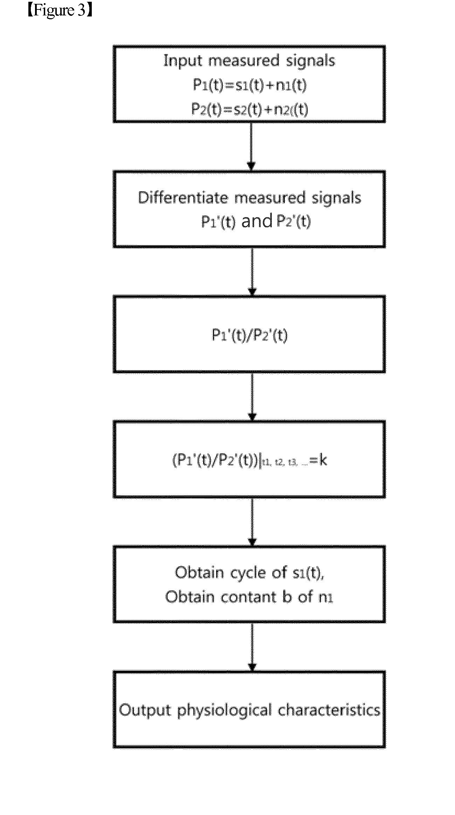

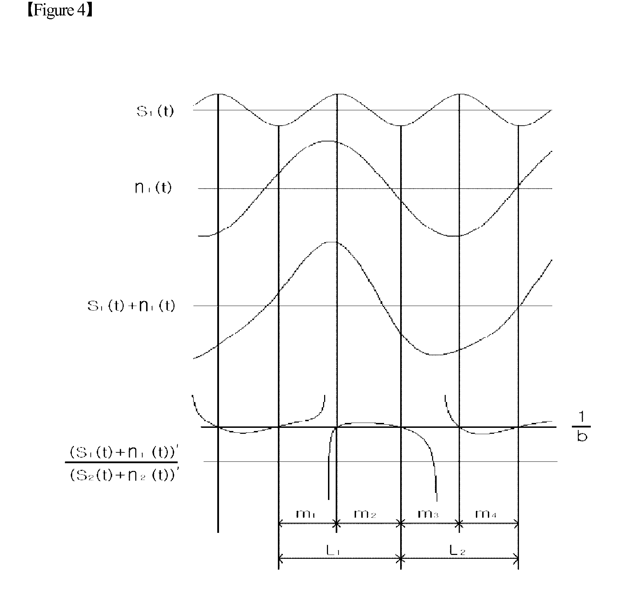

Next, referring to Figs. 3 and 4, as one embodiment of the method for processing a physiological signal according to the invention, a method of obtaining information about the main signal containing data of physiological characteristics, in particular, an exemplary method of extracting a cycle of the main signal will be described.

In this embodiment, the method for processing a physiological signal for measuring physiological characteristics of a living organism includes: step (a) dividing $P_1'(t)$, which is obtained by differentiating one signal function (hereinafter, referred to as $P_1(t)$) between two signal functions ($P(t)$) having different wavelength bands and representing the physiological characteristics of the living organism, by $P_2'(t)$ obtained by differentiating the other signal function (hereinafter, referred to as $P_2(t)$); and step (b) obtaining a constant b of $n_1(t)$ and the cycle of the function $s_1(t)$. Here, the step (b) is processed using Math Formula 1 as follows. The steps (a) and (b) can be carried out by the processor.

[Math Formula 1]

$$(P_1'(t)/P_2'(t))|_{t=t1, t2, t3,...} = k.$$

That is, the function obtained in step (a) may be represented by Math Formula 2 as follows:

[Math Formula 2]

$$(P_1'(t)/P_2'(t)) = (s_1'(t)+n_1'(t))/(s_2'(t)+n_2'(t)).$$

Thus, the Math Formula 2 may be represented by $(s_1'(t)+n_1'(t))/(as_1'(t)+bn_1'(t))$, and if $s_1'(t)$ obtained by differentiating the function $s_1(t)$ is 0 (Zero), the value of the Math Formula 2 becomes $(1/b)$, and $(1/b)=k$ is established under the same conditions.

A signal processor, which processes a physiological signal caused by the physiological characteristics such as oxygen saturation and the like, generates the function $P_1(t)$ from one signal (e.g., an infrared signal) of the two measured signals input to the signal processor and generates the function $P_2(t)$ from the other signal (e.g., red light signal). It should be understood that the functions $P_1(t)$ and $P_2(t)$ are not limited to the above description. Alternatively, the function $P_1(t)$ may be a function for a red light signal and the function $P_2(t)$ may be a function for an infrared signal.

Further, in measurement of the pulse oxygen saturation, $s_1(t)$ and $s_2(t)$ may be signal functions by arterial blood, and $n_1(t)$ and $n_2(t)$ may be signal functions by noise factors such as venous blood or the like.

Next, $P_1(t)$ and $P_2(t)$ are respectively differentiated to obtain the functions $P_1'(t)$ and $P_2'(t)$, and one of the functions $P_1'(t)$ and $P_2'(t)$ is used to divide the other function, thereby obtaining the Math Formula 2 described above.

Further, to extract the cycle of the function $s_1(t)$ at the step (b), if k of the Math Formula 1 is extracted such that the values of variable t allowing the function $P_1'(t)/P_2'(t)$ defined in Math Formula 2 to have the same value can satisfy the condition of including a value repeated in a constant pattern or cycle, a constant b of $n_1(t)$ and the cycle of the function $s_1(t)$ can be obtained.

Referring to Fig. 4, a model for extracting the cycle of the function $s_1(t)$ will be described in more detail. In a graph according to the function of Math Formula 2 having an axis of abscissa representing time t, if intersecting points between a virtual transverse line (k) and the graph of the Math Formula 2 include points repeated in a certain pattern when the virtual transverse line (k) is horizontally shifted in a vertical direction, an inverse of a value on the virtual transverse line (k) becomes k of Math Formula 1.

At this time, according to the Math Formula 2, the values t of the intersecting points between the virtual transverse line (k) and the function of the Math Formula 2 include the values t of coordinates (valleys and peaks of the function $s_1(t)$) where $s_1'(t)$ obtained by differentiating the function $s_1(t)$ becomes 0 (zero), and thus a distance between the valleys or between the peaks of the function $s_1(t)$ is the cycle of the function $s_1(t)$.

Therefore, if the intersecting points between the virtual transverse line (k) and the graph of Math Formula 2 include points repeated at regular intervals with at least one point therebetween, or if the transverse line (k) is determined such that the intersecting points between the virtual transverse line (k) and the graph of Math Formula 2 can include a pair of points (e.g., one corresponding to the valley of the function $s_1(t)$ and the other corresponding to the peak of the function $s_1(t)$) repeated at regular intervals, and the cycle of the points repeated at regular intervals with at least one point therebetween or the cycle of a pair of points repeated at regular intervals is extracted, the cycle of the points becomes the cycle of the function $s_1(t)$.

In more detail, with reference to Fig. 4, if the position of the transverse line (k) is determined by shifting the transverse line (k) up or down such that the intersecting points between the transverse line (k) and the function of Math Formula 2 can have points repeated at regular intervals ($L_1=L_2=...$), $L_1$ becomes the cycle of $s_1(t)$.

Alternatively, if the position of the transverse line (k) is determined by shifting the transverse line (k) up or down such that the intersecting points between the transverse line (k) and the function of Math Formula 2 can have a pair of points repeated at regular intervals ($m_1$ or $m_2$), the cycle of repeating the pair of points becomes the cycle of $s_1(t)$. Here, $m_1=m_3=...$ and, $m_2=m_4=...$.

Further, if the position of the transverse line (k) is determined such that a ratio ($m_1/m_2$) of distances from one of the intersecting points between the transverse line (k) and the function of Math Formula 2 to a certain leftward point and to a certain rightward point can have periodic characteristics, it is possible to extract the constant b of the function $n_1(t)$ and the cycle of the function $s_1(t)$. That is, if ($m_1/m_2$)=($m_3/m_4$)=..., it is possible to extract the cycle of $s_1(t)$.

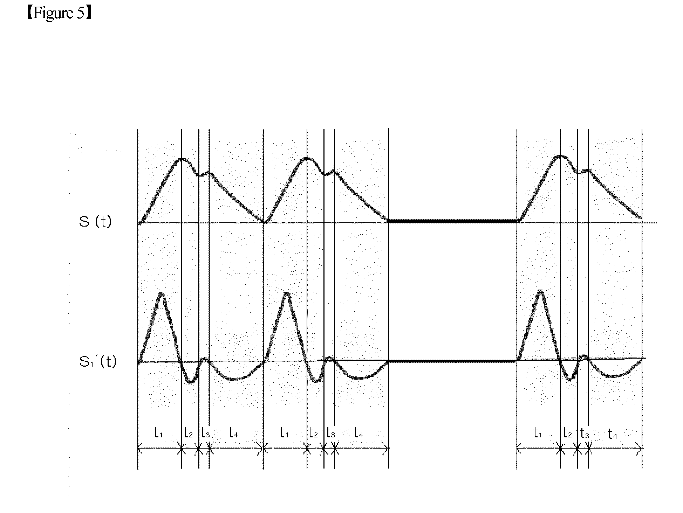

Further, as shown in the graph of Fig. 5, if the position of the transverse line (k) is determined by shifting the transverse line (k) up or down such that the intersecting points between the transverse line (k) and the function of Math Formula 2 can have points repeated in a reference pattern that is set to have a pattern of time t where a value of the differentiated signal $s_1'(t)$ of one cycle including no noise $n_1(t)$ becomes 0 (Zero), it is possible to extract the constant b of the function $n_1(t)$ and the cycle of the function $s_1(t)$.

In this embodiment, the method of extracting the cycle of the function $s_1(t)$ is based on the fact that the value of Math Formula 2 becomes constant at a point (the valley and the peak of the function $s_1(t)$) where the function $s_1'(t)$ becomes 0 (zero). It should be noted that the method of extracting the cycle may be modified in various ways without departing from the spirit of the present invention.

On the contrary, it is possible to extract the constant b and the cycle of the function $s_1(t)$ or $s_2(t)$ by the afore-mentioned method even though the function $P_2'(t)$ is divided by the function $P_1'(t)$ as shown in Math Formula 3 as follows. Since the functions $s_1(t)$ and $s_2(t)$ have the same cycle, it is possible to derive the constant b and the cycle of the main signal 10 of the two measured signals when one of the two functions is extracted.

[Math Formula 3]

$$(P_2'(t)/P_1'(t))=(1/k)=(s_2'(t)+n_2'(t))/(s_1'(t)+n_1'(t))=(as_1'(t)+bn_1'(t))/(s_1'(t)+n_1'(t))$$

$$= (s_2'(t)+n_2'(t))/((s_2'(t)/a)+(n_2'(t)/b))$$

In addition, the cycles of the main signals are detected to ascertain the foregoing physiological characteristics and output the same.

Further, when the pattern of points, at which the value of the signal $s_1'(t)$ including no noise $n_1(t)$ is 0 (Zero), is known, Math Formula 1 may be used to obtain the constant b of the function $n_1(t)$ and the cycle of the function $s_1(t)$.

Further, after the constant b is extracted at step (b), a reference waveform of the signal s(t) may be extracted using Math Formula 4 as follows:

[Math Formula 4]

$$P_1(t)-P_2(t)/b=s_1(t)+n_1(t)-(as_1(t)+bn_1(t))/b=(1-a/b)s_1(t).$$

In the method according to the invention, a signal cycle of desired data on a user, that is, data from which a noise component has been removed, can be extracted through a simple process, thereby simplifying the method for detecting physiological characteristics and thus reducing load applied to a signal processor for processing the physiological signal.

Further, the method according to the invention may reduce a period of time taken in processing the physiological signal, and load applied to devices constituting the signal processor, thereby reducing a heat generation rate of the biological signal sensor, such as a pulse oximeter, while extending lifespan of the devices and decreasing production costs.

Although some embodiments have been described herein, it will be understood by those skilled in the art that these embodiments are provided for illustration only, and that various modifications, changes, alterations and equivalent embodiments can be made without departing from the scope of the invention. Therefore, the scope and spirit of the invention should be defined only by the accompanying claims and equivalents thereof.

[Industrial Applicability]

The present invention is effectively applicable to various medical instruments such as a device for detecting biological characteristics for example oxygen saturation. According to the present invention, detection process of the physiological characteristics can be simplified.

**Claims**

1.  A method for processing a physiological signal to measure physiological characteristics of a living organism, comprising:

    (a) dividing $P_1'(t)$, which is obtained by differentiating one signal function $P_1(t)$ between two signal functions having different wavelength bands and representing the physiological characteristics of the living organism, by $P_2'(t)$ obtained by differentiating the other signal function $P_2(t)$; and
    (b) obtaining a constant b of the following function $n_1(t)$ and a cycle of the following function $s_1(t)$ through Math Formula 1.

    [Math Formula 1]

    $$(P_1'(t)/P_2'(t))|_{t=t1, t2, t3,...} = k,$$

    (where $P_1(t)=s_1(t)+n_1(t)$, $P_2(t)=s_2(t)+n_2(t)$, $as_1(t)=s_2(t)$, $bn_1(t)=n_2(t)$, a, b and k are constants, t is time, and $n_1(t)$ and $n_2(t)$ are signal functions due to noise when measuring the physiological signal).

2.  The method according to claim 1, wherein the step of obtaining a constant b of the function $n_1(t)$ and a cycle of the function $s_1(t)$ comprises obtaining the constant b of the function $n_1(t)$ and the cycle of the function $s_1(t)$ by setting the value k of Math Formula 1 such that a variable t allowing the function $P_1'(t)/P_2'(t)$ to have the same value can satisfy a condition of including a value repeated at a regular cycle.

3.  The method according to claim 1, wherein the step of obtaining a constant b of the function $n_1(t)$ and a cycle of the function $s_1(t)$ comprises obtaining the constant b of the function $n_1(t)$ and the cycle of the function $s_1(t)$ by setting the value k of Math Formula 1 so as to satisfy a condition that intersecting points between a transverse line (k) parallel to an axis (t) of abscissa of the function $P_1'(t)/P_2'(t)$ and the function $P_1'(t)/P_2'(t)$ include intersecting points repeated at regular intervals with at least one intersecting point therebetween.

4.  The method according to claim 1, wherein the step of obtaining a constant b of the function $n_1(t)$ and a cycle of the function $s_1(t)$ comprises obtaining the constant b of the function $n_1(t)$ and the cycle of the function $s_1(t)$ by setting the value k of Math Formula 1 so as to satisfy a condition that intersecting points between a transverse line (k) parallel to an axis (t) of abscissa of the function $P_1'(t)/P_2'(t)$ and the function $P_1'(t)/P_2'(t)$ include intersecting points repeated in a certain pattern.

5.  The method according to claim 1, wherein the step of obtaining a constant b of the function $n_1(t)$ and a cycle of the function $s_1(t)$ comprises obtaining the constant b of the function $n_1(t)$ and the cycle of the function $s_1(t)$ by setting the value k of Math Formula 1 so as to satisfy a condition that intersecting points between a transverse line (k) parallel to an axis (t) of abscissa of the function $P_1'(t)/P_2'(t)$ and the function $P_1'(t)/P_2'(t)$ include intersecting points of a preset pattern.

6.  The method according to any one of claims 1 to 5, wherein Math Formula 1 used in extracting the constant b of the function $n_1(t)$ and the cycle of the function $s_1(t)$ is obtained based on that $s_1'(t)$ obtained by differentiating the function $s_1(t)$ is 0 (zero).

7.  The method according to claim 1, wherein the physiological characteristics comprise pulse oxygen saturation.

8.  The method according to claim 1, wherein the physiological characteristics are measured by an infrared signal and a red light signal.

9.  A method for processing a physiological signal to measure physiological characteristics of a living organism, comprising:

    (a) dividing $P_1'(t)$, which is obtained by differentiating one signal function $P_1(t)$ between two signal functions having different wavelength bands and representing the physiological characteristics of the living organism, by $P_2'(t)$ obtained by differentiating the other signal function $P_2(t)$;
    (b) obtaining a constant b of the following function $n_1(t)$ and a cycle of the following function $s_1(t)$ through Math

Formula 1; and

(c) extracting a reference waveform of the function $s_1(t)$ through Math Formula 4.

[Math Formula 1]

$$(P_1'(t)/P_2'(t))|_{t=t1,\,t2,\,t3,\ldots} = k,$$

(where $P_1(t)=s_1(t)+n_1(t)$, $P_2(t)=s_2(t)+n_2(t)$, $as_1(t)=s_2(t)$, $bn_1(t)=n_2(t)$, a, b and k are constants, t is time, and $n_1(t)$ and $n_2(t)$ are signal functions due to noise when measuring the physiological signal)

[Math Formula 4]

$$P_1(t)-P_2(t)/b=s_1(t)+n_1(t)-(as_1(t)+bn_1(t))/b=(1-a/b)s_1(t)$$

【Figure 1】

【Figure 2】

【Figure 3】

【Figure 4】

【Figure 5】

| | | |
|---|---|---|
| **INTERNATIONAL SEARCH REPORT** | International application No. | |
| | **PCT/KR2013/005835** | |

**A. CLASSIFICATION OF SUBJECT MATTER**

*A61B 5/02(2006.01)i, A61B 5/1477(2006.01)i, G06F 19/00(2011.01)i*

According to International Patent Classification (IPC) or to both national classification and IPC

**B. FIELDS SEARCHED**

Minimum documentation searched (classification system followed by classification symbols)
A61B 5/02; H03M 7/00; G06F 17/17; A61B 5/0452; A61B 5/0402; A61B 8/00; A61B 5/00; A61B 5/1477; G06F 19/00

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: biological signal processing, signal function differential, cycle acquisition

**C. DOCUMENTS CONSIDERED TO BE RELEVANT**

| Category* | Citation of document, with indication, where appropriate, of the relevant passages | Relevant to claim No. |
|---|---|---|
| A | KR 10-2008-0030189 A (INDUSTRY ACADEMIC COOPERATION FOUNDATION, HALLYM UNIVERSITY) 04 April 2008<br>See paragraphs [0017]-[0035]. | 1-9 |
| A | JP 07-194565 A (PACESETTER AB) 01 August 1995<br>See paragraphs [0024]-[0028]. | 1-9 |
| A | KR 10-2009-0009080 A (FREESCALE SEMICONDUCTOR, INC.) 22 January 2009<br>See paragraphs [0019]-[0058]. | 1-9 |
| A | KR 10-2007-0013981 A (SAMSUNG MEDISON CO.,LTD.) 31 January 2007<br>See page 3, line 17-page 4, line 7. | 1-9 |

☐ Further documents are listed in the continuation of Box C. ☒ See patent family annex.

| | | | |
|---|---|---|---|
| \* | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 OCTOBER 2013 (29.10.2013) | **01 NOVEMBER 2013 (01.11.2013)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea | |
| Facsimile No. 82-42-472-7140 | Telephone No. |

Form PCT/ISA/210 (second sheet) (July 2009)

INTERNATIONAL SEARCH REPORT
Information on patent family members

International application No.

**PCT/KR2013/005835**

| Patent document cited in search report | Publication date | Patent family member | Publication date |
|---|---|---|---|
| KR 10-2008-0030189 A | 04/04/2008 | NONE | |
| JP 07-194565 A | 01/08/1995 | DE 69420235 D1 | 30/09/1999 |
| | | DE 69420235 T2 | 30/03/2000 |
| | | EP 0656219 A1 | 07/06/1995 |
| | | EP 0656219 B1 | 25/08/1999 |
| | | SE 9304029 D0 | 03/12/1993 |
| | | US 05556419 A | 17/09/1996 |
| KR 10-2009-0009080 A | 22/01/2009 | CN 101164239 A0 | 16/04/2008 |
| | | CN 101164239 B | 18/08/2010 |
| | | CN 101167254 A0 | 23/04/2008 |
| | | CN 101167254 B | 06/10/2010 |
| | | JP 05-017257 B2 | 05/09/2012 |
| | | JP 2008-539469 A | 13/11/2008 |
| | | US 2006-0244640 A1 | 02/11/2006 |
| | | US 2006-0244644 A1 | 02/11/2006 |
| | | US 7109906 B1 | 19/09/2006 |
| | | US 7327288 B2 | 05/02/2008 |
| | | US 7561076 B2 | 14/07/2009 |
| | | WO 2006-119025 A1 | 09/11/2006 |
| | | WO 2007-114828 A1 | 11/10/2007 |
| KR 10-2007-0013981 A | 31/01/2007 | JP 04-899439 B2 | 21/03/2012 |
| | | JP 2007-029703 A | 08/02/2007 |
| | | US 2007-0038101 A1 | 15/02/2007 |
| | | US 2009-0318811 A1 | 24/12/2009 |
| | | US 8012093 B2 | 06/09/2011 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6002952 A
- US 5490505 A